# FASCICULE DE BREVET EUROPEEN

(11) **EP 2 225 546 B1**
(45) Date de publication et mention de la délivrance du brevet: **27.04.2011**
(21) Numéro de dépôt: 08866713.4
(22) Date de dépôt: 24.12.2008
(51) Int. Cl.: G01N 9/02, G01F 17/00

(54) **PYCNOMETRE**
PYKNOMETER
PYCNOMETER

(30) Priorité: 27.12.2007 FR 0760396
(43) Date de publication de la demande: 08.09.2010
(73) Titulaire: Commissariat à l'Énergie Atomique et aux Énergies Alternatives, 75015 Paris (FR)
(72) Inventeur: JORION, Frédéric, F-30200 Sabran (FR); BRENNEIS, Christophe, F-30290 Saint Victor La Coste (FR); POLLE, Flavie, F-33370 Yvrac (FR)
(74) Mandataire: Ilgart, Jean-Christophe
(86) Numéro de dépôt international: PCT/EP2008/068284
(87) Numéro de publication internationale: WO 2009/083566

(56) Documents cités:
- EP-A- 0 720 011
- US-A- 4 095 473
- US-A- 5 074 146

## Description

L'invention représente un pycnomètre, c'est-à-dire un dispositif de mesure de volume d'un corps appelé l'échantillon qu'on place dans une chambre de mesure qui est mise sous pression gazeuse. On organise ensuite une détente partielle du gaz vers une chambre de détente qu'on fait communiquer avec la chambre à l'échantillon. La mesure du changement de pression permet de déduire le volume de l'échantillon à condition de connaître les volumes des deux chambres. Ces procédés sont particulièrement utiles pour déterminer le volume réel des corps poreux, que les gaz peuvent bien pénétrer. Complétée par une mesure de la masse de l'échantillon, l'indication de volume peut permettre de déterminer la masse volumique ou la densité du corps composant l'échantillon.

Les pycnomètres sont toutefois sujets à des imprécisions provenant notamment des capteurs de pression, des estimations des volumes de mesure et de l'existence de volumes morts affectés à la communication entre les chambres et l'extérieur. Les difficultés sont encore plus importantes avec des échantillons radioactifs dont les radiations endommagent vite les capteurs de pression usuels et provoquent des échauffements qui altèrent la pression des gaz et faussent les mesures. Les appareils usuels ne permettent pas non plus des manipulations dans les cellules blindées où se trouvent les échantillons.

US-A-5 074 146, US-A-4 095 473 et EP-A-0 720 011 décrivent des pycnomètres présentant ces insuffisances. Le premier d'entre eux sert à former le préambule de la revendication principale.

L'objet de l'invention est d'améliorer les pycnomètres existants sous ces aspects, afin de parvenir à des mesures plus précises et à des manipulations sans risque même pour des échantillons radioactifs.

Sous une forme générale, l'invention concerne un pycnomètre comprenant deux chambres communicantes, dont une chambre d'échantillon et une chambre de détente, et des vannes pour faire communiquer les chambres entre elles et avec l'extérieur ou les isoler, les chambres étant creusées dans un même bloc par usinage, caractérisé en ce que deux capteurs de pression sont respectivement associés aux chambres, les capteurs de pression sont montés sur le bloc, entourés d'un blindage aux radiations, le capteur de pression associé à la chambre d'échantillon est raccordé à ladite chambre par un conduit coudé, et il existe un capteur de température de la chambre d'échantillon.

L'appareil se présente sous une forme massive, à la fois unitaire et solide, qui permet de le manipuler facilement. Le blindage et le coude du conduit permettent d'isoler les capteurs des radiations, en évitant de les exposer directement à la chambre à l'échantillon. Le capteur de température permet d'appliquer une correction. Enfin, le creusement des chambres dans le bloc assure la précision de leur volume.

D'après un autre perfectionnement, les chambres communiquent entre elles, avec l'extérieur, avec le capteur de température et avec les capteurs de pression exclusivement par des conduites consistant en des perçages usinés dans le bloc, et éventuellement par des plans de joint séparant des parties du bloc qui sont assemblées entre elles avec un serrage ou boulonnage.

Ces perçages usinés dans le corps étant très fins, les volumes morts du réseau de conduits seront réduits et pourront d'ailleurs être déterminés avec précision.

D'après un autre perfectionnement, les vannes comprennent des pistons coulissant dans des alésages du bloc, les alésages comprenant un fond dans lequel débouchent deux conduits.

Cette mesure aussi réduit les volumes morts du réseau, assure la précision des mesures et permet une commande facile.

Il est avantageux encore, afin d'améliorer la manipulation du pycnomètre, que le corps comprenne des dispositifs d'actionnement des vannes et un dispositif d'ouverture et de fermeture de la chambre à l'échantillon.

Enfin, la précision des mesures peut aussi être améliorée si l'appareil comprend un dispositif de variation de volume dans la chambre de détente.

On va maintenant décrire l'invention sous tous ses aspects au moyen d'une réalisation particulière associée aux figures :
- la figure 1 est un schéma de fonctionnement d'un pycnomètre, et
- les figures 2, 3, 4 et 5 illustrent la réalisation plus particulièrement proposée ici : la figure 2 est une vue extérieure, la figure 3 une coupe longitudinale, la figure 4 une vue partielle illustrant des positions de principaux éléments et le réseau de conduits les desservant, et la figure 5 est une vue extérieure complète.

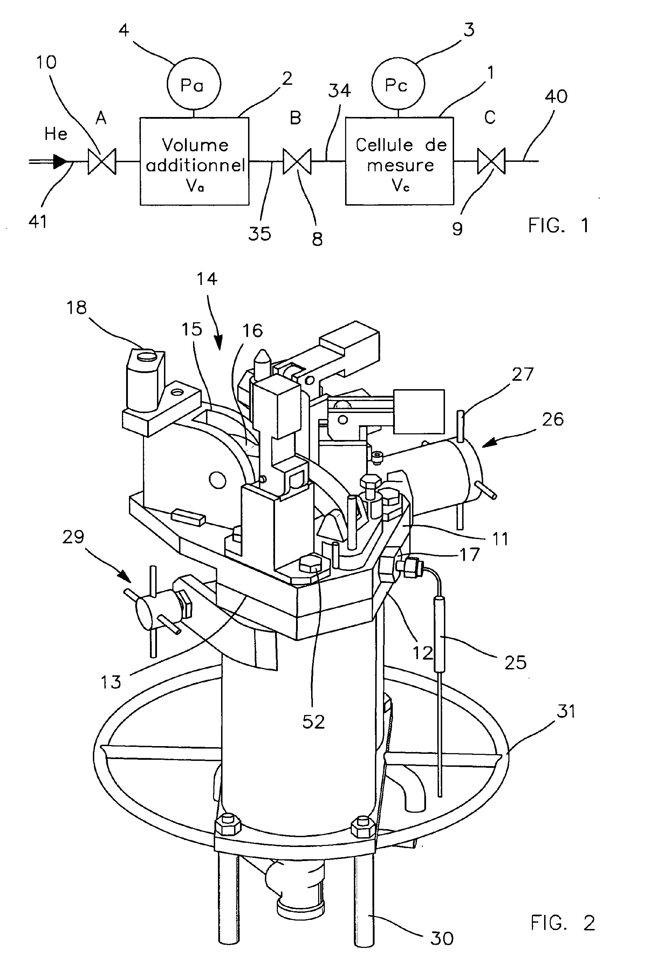

D'après la figure 1, le système comprend une chambre d'échantillon 1, une chambre de détente 2, deux capteurs de pression 3 et 4 respectivement associés aux chambres 1 et 2. Un conduit 34 de communication entre la chambre 1, le capteur de pression 3 et les vannes 8 et 9, et un conduit 35 de communication entre la chambre 2, le capteur de pression 4 et les vannes 8 et 10 forment le réseau de gaz. Les conduits 40 et 41 permettent respectivement la mise à l'air de la vanne 9 et l'entrée de gaz dans l'appareil. Si on appelle V le volume d'un échantillon placé dans la chambre d'échantillon 1, V1 et V2 les volumes des chambres 1 et 2, P1 et P2 les pressions dans les chambres 1 et 2 avant la détente et PF la pression commune dans les chambres après la mise en communication et la détente, la formule suivante donne le volume V de l'échantillon : V=V1+(PF-P2/PF-P1).V2.

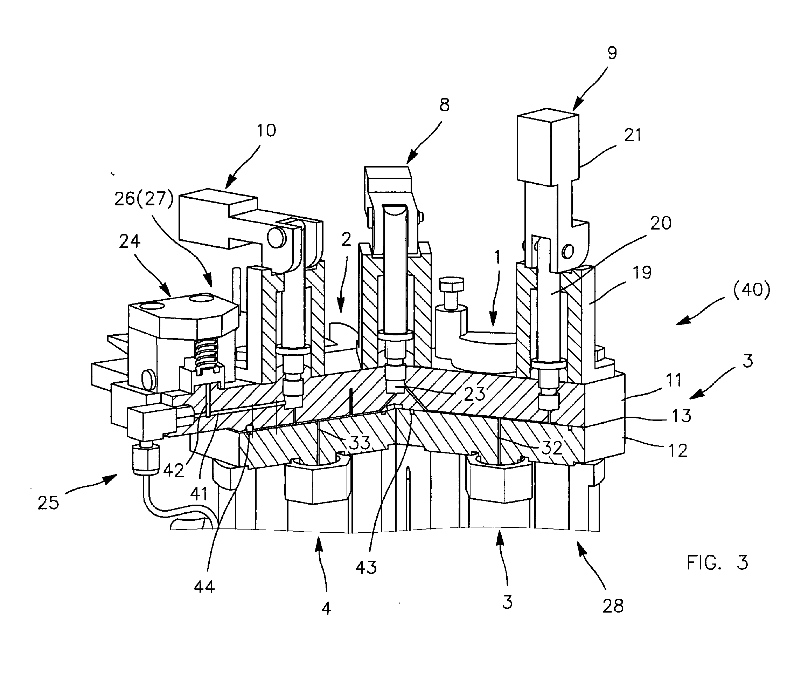

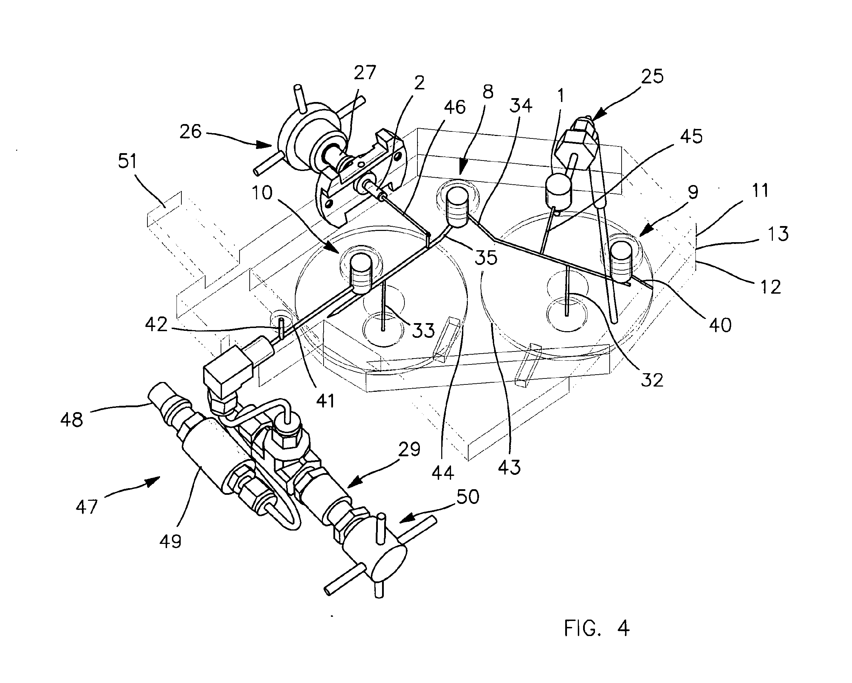

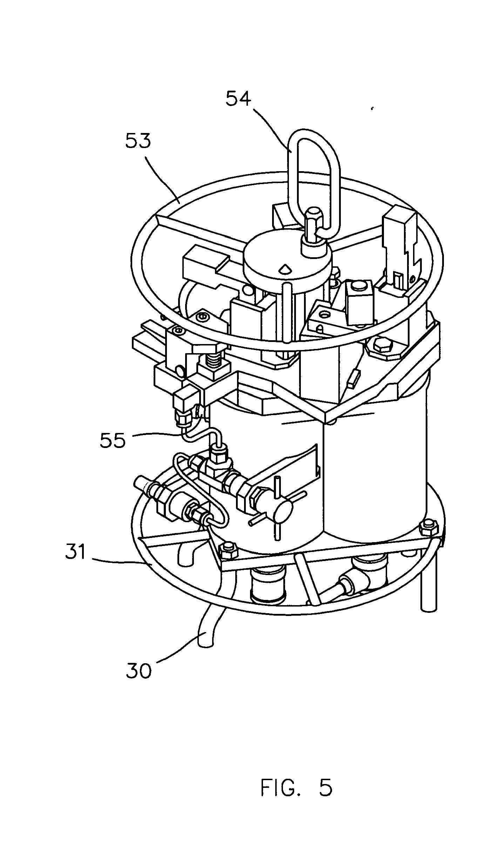

On passe maintenant aux commentaires des figures 2, 3, 4 et 5. Le coeur de l'appareil est un bloc 36 composé d'une culasse supérieure 11 et d'une culasse inférieure 12 pressées l'une contre l'autre par un plan de joint 13. Les équipements de l'appareil apparaissent principalement à la surface supérieure de la culasse supérieure 11, mais certains sont montés à la périphérie du bloc 36, autour du plan de joint 13, et à la surface inférieure de la culasse inférieure 12.

La chambre d'échantillon 1 et la chambre de détente 2 sont usinées dans la culasse supérieure 11 ; la chambre d'échantillon 1 s'ouvre sur la face supérieure de la culasse supérieure 11 mais peut être fermée par le mouvement d'une bride 14 comprenant une chape 15 montée sur la culasse supérieure 11, un levier 16 tournant sur la chape 15, une porte 17 articulée sur la culasse supérieure est plaquée sur la culasse supérieure, au droit de la chambre d'échantillons 1, à l'aide du levier 16 et une manivelle 18 de verrouillage du levier 16.

Les vannes 8, 9 et 10 comprennent un canon 19 boulonné à la culasse supérieure 11, une tige 20 glissant dans le canon 19, une manivelle 21 montée au sommet du canon 19 faisant coulisser le piston 20 pénétrant dans un alésage 23 de la culasse supérieure 11, pour assurer à chaque fois l'obstruction ou la communication des conduits 34 et 35 ainsi qu'on le verra plus en détail. Les vis 52 de fixation des vannes 8, 9 et 10 assurent le pressage des culasses 11 et 12 l'une contre l'autre au plan de joint 13. La culasse supérieure 11 porte encore un évent 24 pouvant être actionné par une manette sur sa face supérieure et, sur la périphérie, un thermocouple 25 sous la chambre d'échantillon 1 et un dispositif de réglage de volume 26 de la chambre de détente 2 comprenant une vis micrométrique 27 y pénétrant.

La culasse inférieure 12 porte essentiellement les capteurs de pression 3 et 4, un blindage 28 aux radiations les entourant, et un régulateur de débit 29 muni d'un cabestan de manoeuvre 50. Le blindage 28 est muni d'un raccord 55 permettant de connecter une arrivée de gaz afin de ventiler les capteurs de pression dans le but de les refroidir. Le blindage 28 porte un anneau de guidage 31, pour faciliter la manipulation de l'appareil à l'horizontale dans le tunnel. Un anneau de guidage identique 53, mais démontable est mis en place lors des manutentions à l'horizontale, afin d'assurer un guidage et une protection maximale de l'appareil. Après ouverture de la porte intérieure du tunnel, l'appareil est saisi à l'aide d'un moyen de manutention existant dans la cellule blindée (palan) par son anneau de levage 54. Il est alors posé sur le sol de la cellule et repose sur 4 pieds 30. L'anneau de levage 54 et l'anneau de guidage 53 sont ensuite démontés.

On va maintenant décrire l'agencement interne du bloc 36. Des conduits 32 et 33 sont percés à de fins rayons à travers la culasse inférieure 12 et mènent aux capteurs de pression 3 et 4. D'autres conduits fins 34 et 35 sont percés dans la culasse supérieure 11 entre sa face inférieure et les alésages 23 des vannes, 34 menant de la vanne 8 à la vanne 9 et 35 de la vanne 8 à la vanne 10. Deux fins conduits 45 et 46 sont encore percés entre la face inférieure de la culasse supérieure 11 et, respectivement, la chambre d'échantillon 1 et la chambre de détente 2. Un conduit 40 est creusé dans la culasse supérieure 11 entre le côté de l'alésage 23 de la vanne 9 et l'extérieur et servant à l'éventement aval ; un conduit 41 est creusé dans la culasse supérieure 11 entre le côté de l'alésage 23 et de la vanne 10 et l'extérieur et se raccorde à un dispositif d'arrivée de gaz 47 pouvant comprendre un raccord 48 vissé sur une partie 49 liée au blindage des capteurs de pression assurant une rigidité lors des connexions déconnexions de la tuyauterie d'arrivée de gaz et le régulateur de débit 29 ; enfin, le réseau comprend un conduit 42 bifurquant du conduit 41 et joignant l'évent 24. Cet évent s'ouvre en plaquant, à l'aide de la pince du télémanipulateur de la cellule, sa tige de manoeuvre sur une excroissance 51 de la culasse supérieure. Tous ces derniers conduits sont eux aussi percés à de fins diamètres.

Les conduits 34 et 45 débouchent dans une étendue du plan de joint 13 qui est englobée par un joint d'étanchéité 43 logé entre les culasses 11 et 12. De même, les conduits 35 et 46 débouchent dans une étendue du plan de joint 13 qui est englobée par un autre joint d'étanchéité 44. Les conduits 34 et 35 qui servent de conduits de liaison entre des conduits creusés dans les deux culasses 11 et 12 possèdent chacun une portion creusée dans la face inférieure de la culasse supérieure et s'ouvrent donc dans le plan de joint 13, ce qui rend les liaisons plus faciles à établir. Ces portions sont incluses dans le pourtour des joints d'étanchéité 43 et 44 qui évitent donc les fuites par le plan de joint 13.

Des volumes morts de l'appareil comprennent ainsi les perçages usinés dans la masse du bloc 36, dont le volume est faible grâce à la finesse des perçages, les étendues englobées par les joints d'étanchéité 43 et 44, dont les volumes sont aussi faibles grâce au peu de jeu du plan de joint 13, et les volumes des alésages 23, qui sont faibles eux aussi grâce au petit mouvement qui est nécessaire pour que les pistons 20 établissent la communication entre les paires de conduits débouchant au fond des alésages 23 (34 et 35) et au bas de leur paroi latérale (35, 40 et 41). Ces volumes morts sont d'ailleurs déterminés avec précision grâce à un usinage de qualité.

Les capteurs de pression 3 et 4 sont éloignés de la chambre d'échantillon 1 et ne sont donc pas susceptibles d'être endommagés par des radiations, d'abord grâce au blindage 28 et ensuite grâce aux coudes entre les conduits 32, 34 et 4 entre la chambre d'échantillon 1 et le capteur de pression 3 associé, ce qui interdit l'irruption du rayonnement ; le trajet jusqu'à l'autre capteur de pression 4 est évidemment encore plus sinueux.

Le thermocouple 25 est en communication presque directe avec la chambre d'échantillon 1 par un perçage 38 de communication creusé dans la culasse supérieure 11, et qui a été rebouché pour être hermétique aux gaz. La mesure de la température du gaz est ainsi précise.

Le mode d'emploi de ce pycnomètre et la mesure sont classiques : l'échantillonnage est mis dans la chambre 1, qu'on referme ensuite, et le volume de la chambre de détente 2 est ajusté ; une bouteille de gaz (non représentée) est branchée au raccord 48, et son gaz emplit les deux chambres 1 et 2, la vanne 9 étant fermée ; la mise en pression des chambres est régulée par le limiteur de débit 29 ; lorsque la pression désirée est atteinte, la vanne 8 est fermée pour isoler la chambre d'échantillon 1, la surpression dans la chambre de détente 2 s'écoule en actionnant l'évent 24 et la vanne 10 est alors fermée ; la vanne 8 est ensuite ouverte, et la mesure commence. Les mesures de pression sont toutefois, de préférence, enregistrées continuellement pendant toute l'expérience de même que les mesures de température de manière à corriger l'évolution de la pression pour en soustraire la portion due à l'évolution de la température ; cela peut être fait en appliquant la formule de définition des gaz parfaits.

Les manivelles 18 et 21 se prêtent à une mise en service de l'appareil par des moyens de télémanipulation dont la dextérité est donc rudimentaire, et de même la vis micrométrique et le limiteur de débit 29 peuvent être tournées facilement grâce à la mise en place des cabestans 27 et 50 sur leur bouton de manoeuvre.

## Revendications

1. Pycnomètre comprenant deux chambres (1, 2) communicantes, dont une chambre d'échantillon (1) et une chambre de détente (2), et des vannes (8, 9, 10) pour faire communiquer les chambres entre elles et avec l'extérieur ou les isoler, les chambres étant creusées dans un même bloc (36) par usinage, **caractérisé en ce que** deux capteurs de pression (3, 4) sont respectivement associés aux chambres, les capteurs de pression sont montés sur le bloc, entourés d'un blindage (28) aux radiations, les capteurs de pression (3, 4) sont raccordés à ladite chambre d'échantillon (1) par des conduits coudés, et il existe un capteur de température (25) de la chambre d'échantillon.

2. Pycnomètre selon la revendication 1, **caractérisé en ce que** les chambres communiquent entre elles, avec l'extérieur, avec les capteurs de pression exclusivement par des conduites consistant en des perçages usinés dans le bloc.

3. Pycnomètre selon l'une quelconque des revendications 1 ou 2, **caractérisé en ce que** les vannes (8, 9, 10) comprennent des pistons (20) coulissant dans des alésages (23) du bloc, les alésages comprenant chacun un fond dans lequel débouche un des conduits et une paroi latérale au bas de laquelle débouche un autre des conduits.

4. Pycnomètre selon l'une quelconque des revendications 1 à 3, **caractérisé en ce que** le bloc comprend une face unique sur laquelle sont exposés des dispositifs d'actionnement (21) des vannes (8, 9, 10) et un dispositif (14) d'ouverture et de fermeture de la chambre d'échantillon (1).

5. Pycnomètre selon l'une quelconque des revendications 1 à 4, **caractérisé en ce qu'**il comprend un dispositif (26) de variation de volume de la chambre de détente (2).

6. Pycnomètre selon la revendication 1, **caractérisé en ce que** le bloc (36) est composé de parties (11, 12) assemblées entre elles avec un boulonnage et serrage à un plan de joint (13).

7. Pycnomètre selon les revendications 2 et 6, **caractérisé en ce que** certains des conduits (34, 35) comprennent des portions donnant sur le plan de joint (13).

8. Pycnomètre selon la revendication 7, **caractérisé en ce que** lesdits portions donnant sur le plan de joint sont incluses dans le pourtour de joints circulaires (43, 44) établis entre les parties (11, 12) du bloc (36).

9. Pycnomètre selon la revendication 7 ou 8, **caractérisé en ce que** lesdits portions donnant sur le plan de joint (13) se raccordent à d'autres conduits (32 à 37 ; ; 45, 46) percés à travers les blocs entre le plan de joint (13) et soit les chambres (1, 2), soit les capteurs de pression (3, 4), soit les vannes (8, 9, 10).

## Claims

1. Pycnometer comprising two communicating chambers (1, 2), one of which is a sample room (1) and the other an expansion room (2), the chambers being carved out of a single slab (36) during manufacture, and valves (8, 9, 10) to communicate the chambers with each other and with the outside, or to isolate them, **characterized in that** two pressure sensors (3, 4) are respectively associated with the chambers, the pressure sensors are mounted on the slab, surrounded by a radiation-proof shield (28), the pressure sensors (3, 4) are connected to said sample chamber (1) via elbowed conduits, and there is a temperature sensor (25) for the sample chamber.

2. Pycnometer according to Claim 1, **characterized in that** the chambers communicate with each other, with the outside, with the pressure sensors, exclusively via conduits consisting of bores factory-engineered into the slab.

3. Pycnometer according to any one of Claims 1 or 2, **characterized in that** the valves (8, 9, 10) comprise pistons (20) that slide inside bores (23) of the slab, the bores each containing a base to which one of the conduits extends, and a lateral side, at the base of which another conduit extends.

4. Pycnometer according to any one of Claims 1 to 3, **characterized in that** the slab comprises a single side on which the actuating devices (21) of the valves (8, 9. 10) are located, and an opening and closing device (14) for the expansion chamber.

5. Pycnometer according to any one of the Claims 1 to 4, **characterized in that** it comprises a device (26) for varying the volume of the expansion room (2).

6. Pycnometer according to Claim 1, **characterized in that** the slab (36) comprises parts (11 and 12), assembled together via bolting and tightening at a mounting surface (13).

7. Pycnometer according to Claims 2 and 6, **characterized in that** certain conduits (34, 35) comprise portions that extend to the mounting surface (13).

8. Pycnometer according to Claim 7, **characterized in that** said portions extending to the mounting surface are included inside the edge of the circular seals (43, 44) installed between parts (11, 12) of the slab (36).

9. Pycnometer according to Claims 7 or 8, **characterized in that** said portions extending to the mounting surface (13) are connected to other conduits (32 to 37; 45, 46) pierced through the slabs, between the mounting surface (13), and either the chambers (1, 2), the pressure sensors (3, 4), or the valves (8, 9, 10).

## Patentansprüche

1. Pyknometer (System zur Volumen- / Dichtemessung), mit zwei miteinander kommunizierenden Kammern (1, 2), von welchen die eine eine Probenkammer (1) und die andere eine Entspannungskammer (2) darstellt, sowie mit Ventilen (8, 9, 10), um die Kammern miteinander und mit dem Außenraum in Verbindung zu setzen oder sie zu isolieren, wobei die Kammern in ein und demselben Block (36) mittels maschineller Bearbeitung ausgebildet sind, **dadurch gekennzeichnet, dass** zwei Druck-Meßfühler (3, 4) jeweils den Kammern zugeordnet sind, dass die Druckmeßfühler auf dem Block von einer Strahlungsabschirmung (28) umgeben angeordnet sind, dass die Druckmeßfühler (3 , 4) mit der Probenkammer (1) über gekrümmte bzw. gekröpfte Leitungen verbunden sind, und dass ein Meßfühler (25) für die Temperatur der Probenkammer vorgesehen ist.

2. Pyknometer nach Anspruch 1, **dadurch gekennzeichnet, dass** die Kammern untereinander, mit dem Außenraum und mit den Druckmeßfühlern ausschließlich über Leitungen verbunden sind, die aus in dem Block durch Bearbeitung ausgebildeten Bohrungen bestehen.

3. Pyknometer nach einem beliebigen der Ansprüche 1 oder 2, **dadurch** gekenntzeichnet, dass die Ventile (8, 9, 10) in Bohrungen (23) des Blocks gleitbare Kolben (20) aufweisen, und dass die Bohrungen jede jeweils einen Boden, in welchem eine der Leitungen mündet, und eine Seitenwandung aufweisen, in deren unterem Teil eine andere von den Leitungen mündet.

4. Pyknometer nach einem beliebigen der Ansprüche 1 bis 3, **dadurch gekennzeichnet, dass** der Block eine einzige Hauptfläche aufweist, auf welcher Vorrichtungen (2) zur Betätigung der Ventile (8, 9, 10) und eine Vorrichtung zum Öffnen und zum Schließen der Probenkammer (1) angeordnet sind.

5. Pyknometer nach einem beliebigen der Ansprüche 1 bis 4, **dadurch gekennzeichnet, dass** es eine Vorrichtung (26) zur Volumenänderung der Entspannungskammer (2) umfasst.

6. Pyknometer nach Anspruch 1, **dadurch gekennzeichnet, dass** der Block (36) aus Teilen (11, 12) zusammengesetzt ist, die miteinander durch Verschraubung und Festklemmen mit einer Dichtungsebene bzw. -fuge (13) montiert sind.

7. Pyknometer nach den Ansprüchen 2 und 6, **dadurch gekennzeichnet, dass** bestimmte von den Leitungen (34, 35) Bereiche umfassen, die an der Dichtungsfuge bzw. -ebene (13) münden.

8. Pyknometer nach Anspruch 7, **dadurch gekennzeichnet, dass** die in der Dichtungsebene bzw. -fuge mündenden Bereiche im Umfangsverlauf von zwischen den Blockteilen (11, 12) vorgesehenen Kreis- bzw. Ringdichtungen (43, 44) enthalten sind.

9. Pyknometer nach Anspruch 7 oder Anspruch 8, **dadurch gekennzeichnet, dass** die in der Dichtungsebene bzw. -fuge (13) mündenden Bereiche mit anderen Leitungen
(32 bis 37; 45, 46) verbunden sind, die durch die Blöcke hindurch zwischen der Dichtungsebene bzw. -fuge (13) und entweder den Kammern (1, 2) oder den Druckmeßfühler (3, 4) oder den Ventilen (8, 9, 10) gebohrt sind.
